# EUROPEAN PATENT SPECIFICATION

(11) **EP 2 340 080 B1**
(45) Date of publication and mention of the grant of the patent: **05.07.2017**
(21) Application number: 09812297.1
(22) Date of filing: 04.09.2009
(51) Int. Cl.: A61N 1/04

(54) **ELECTRODE CHAIN**
ELEKTRODENKETTE
ELECTRODE EN FORME DE CHAÎNE

(30) Priority: 08.09.2008 US 206557
(43) Date of publication of application: 06.07.2011
(73) Proprietor: Axelgaard Manufacturing Co., Ltd, Fallbrook, CA 92028 (US)
(72) Inventor: AXELGAARD, Jens, Fallbrook CA 92028 (US)
(74) Representative: Kohler Schmid Möbus Patentanwälte
(86) International application number: PCT/US2009/056069
(87) International publication number: WO 2010/028265

(56) References cited:
- US-A- 4 365 634
- US-A- 4 926 879
- US-A- 5 445 537
- US-A- 5 445 537
- US-A- 5 785 040
- US-A1- 2007 238 944
- US-A1- 2007 238 944

## Description

The present invention generally relates to electrodes and, more particularly, electrodes suitable for transcutaneous nerve and/or muscle stimulation and biological signal recording.

Medical electrodes must provide an even electrical distribution to a patient's skin over an entire surface of the electrode to assure proper coupling. Because of the curvaceous nature of the human body, it is apparent that medical electrodes for use thereon must be flexible not only for conformation with a patient's skin contours, but also to accommodate relative movement of the patient's skin.

It is well known that inadequate flexing and shaping of the electrode to a patient's contour can result in an irritation of the patient's skin. Electrical "hot spots" due to uneven electrode-skin contact can result in a rash or a burning sensation. A sensation of burning may be felt by a patient within a few minutes after application of the electrical signals during nerve and/or muscle stimulation, while rash conditions generally take a longer period of time to develop.

In order to provide uniform electrical coupling, heretofore developed electrodes have utilized conductive fabrics and foils in combination with a conductive and flexible adhesive in order to uniformly couple electrical signals to and/or from an electrical lead wire, or connector. A number of electrodes have provided impedance compensation for directing electrical pulses from the lead wire uniformly throughout an electrode, such as, for example, U.S. Patent No. 5,038,796 entitled, ELECTRICAL STIMULATION ELECTRODE WITH IMPEDANCE COMPENSATION, and U.S. Patent No. 5,904,712 CURRENT CONTROLLING ELECTRODE to Axelgaard. U.S. Patent No. 4,736,752 teaches the control of current density across an electrode through the use of conductive ink design areas.

Many prior art electrodes have compromised the flexibility of the electrode in order to provide adequate current densities over the entire contact area of the electrode. Such electrodes typically have utilized a metallic mesh, or foil, to provide conductivity and utilize a conductive gel between the electrode and the patient's skin in order to accommodate the movement therebetween. Such use of foil or mesh often cause burning or hot spots at electrode edges.

US 2007/238944 A1 discloses a medical electrode including a moderately conductive flexible member having a top side and a bottom side with a connector and contact with the conductive flexible member top side for establishing electrical contact with an external apparatus. An oversize non-conductive flexible sheet covers the conductive flexible member top side and the connector and a highly conductive ink pattern is disposed on the conductive flexible member bottom side. A moderately high conductive hydrogel adhesive disposed on the conductive flexible member bottom side and covering the conductive ink pattern is provided for adhering the electrode to a patient's skin. Lateral conductivity and conformability of the electrode is controlled by cutouts in and thickness of the conductive flexible member and/or the conductive adhesive.

The present invention is directed to a medical electrode having a combination of conductive elements, with selected conductivities which enables assembly of the electrode in a manner hereinbefore not possible. More specifically, the present invention is directed to a medical electrode having a connector disposed on a top surface of a conductive member. This enables automated assembly of the electrode as opposed to conventional manual assembly which in turn reduces unit cost while at the same time providing for controlled and even current density. Interconnection of multiple electrodes may be effected through the use of a leadwire having alternating conductive and non-conductive portions.

In addition, mechanical control of lateral conductivity is effected through the use of cutouts in the conductive member and/or conductive adhesive which also adds to the flexibility/conformability of the electrode.

### SUMMARY OF THE INVENTION

The present invention is defined by the appended claims. The examples, embodiments, or aspects of the present description that do not fall within the scope of said claims are merely provided for illustrative purposes and do not form part of the invention.

A medical electrode in accordance with the present invention generally includes a moderately conductive flexible member having a top side and a bottom side with a highly conductive pattern, such as, for example conductive ink, printed or transferred to the member bottom side.

A conductive adhesive of moderately high conductivity is disposed on the flexible member bottom side and covering the conductive pattern for adhering the electrode to a patients' skin.

Importantly, the use of a moderately high conductivity adhesive enables the placement of a connector on the top side of the flexible member while at the same time providing uniform current distribution by the electrode.

In controlling current density, the surface resistivity of the conductive member may be between about 10² and about 10⁶ ohm/cm, the resistivity of the conductive pattern may be between about 0.1 and about 10² ohm and the volume resistivity of the adhesive may be between about 10² and 10⁴ ohm cm. The conductivity of the conductive pattern can be controlled through the use of various grid designs with preselected line widths and spacing as well as thickness and ink compositions.

The connector is disposed over the conductive ink pattern and on the top side of the conductive member, whereas the ink pattern is disposed on the bottom side of the conductive member. This arrangement enables the connectors to be disposed in any selected points within a perimeter of the pattern without affecting current distribution. This flexibility of connector positioning, provided by the present invention, facilitates manufacture of the electrodes. In addition, because the lead wire is not disposed between the conductive pattern and patients' skin, there is no interference with the electrode current distribution as is the case with some prior art electrodes.

A non-conductive flexible sheet may be disposed over the connector on the conductive flexible member top side. The non-conductive flexible sheet preferably has dimensions greater than said conductive flexible member causing an overlap thereof. This arrangement facilitates manufacture and also eliminates the need for precise alignment with the conductive flexible member. It also provides a seal of the gel edge and prevents gel from folding around an edge of the conductive flexible member and attaching itself to clothing, etc.

An adhesive is provided for bonding the non-conductive flexible sheet to the top side of said conductive flexible member and also for securing said connector to said conductive flexible member top side;

In one embodiment of the present invention, the conductive pattern is disposed on the conductive flexible member bottom at a spaced apart distance from a perimeter of the conductive flexible member in order to establish a border between the perimeter of the conductive ink pattern and the conductive flexible member perimeter. This is important in providing controlled "roll off' of electrical current distribution. While even and uniform electrical current density across the electrode is the desired distribution, such current density should not be present at the edge of the electrode since it may cause unwanted stimulation at that site. Thus, it is most desirable to have the current density "roll off" or be reduced to zero over a short distance. The border arrangement in accordance with the present invention provides for such desired current roll off while providing uniform current distribution over the electrode from border to border.

In yet another embodiment of the present invention, the medical electrode includes a moderately conductive flexible member having a top and a bottom side with a plurality of connectors in contact with the conductive member top side for establishing electrical contact with an external apparatus, and a plurality of highly conductive patterns are disposed on the conductive flexible member bottom side where each electrode is electrically isolated from an adjacent electrode.

This feature provides for the advantage of fixed electrode distances which assures proper application of the electrode for optimum patient stimulation or signal recording when multiple electrodes are utilized.

A moderately high conductive adhesive is disposed on the conductive member bottom side and covers the conductive ink patterns for adhering the electrode to a patient's skin.

It should be appreciated that the lead wire may be attached or held in place on the conductive flexible member top side in any manner, and inasmuch as the current distribution across the electrode conductive gel is controlled by the relative conductivities of the flexible member, ink pattern and the adhesive, the connector can be placed anywhere within the borders of the ink pattern as hereinabove noted.

Yet another embodiment of a medical electrode in accordance with the present invention utilizes a moderately conductive flexible member having a topside and a bottom side along with a plurality of highly conductive patterns disposed on the conductive flexible member bottom side.

As in earlier described embodiments, a moderately high conductive adhesive layer is disposed on the conductive flexible member bottom side and covers the conductive patterns for adhering electrode to a patient's skin. In order to control lateral conductivity within the electrode at least one flexible member cutout may be formed in the flexible member for controlling resistivity across the electrode and particularly between the conductive patterns. Lateral conductivity as used in the present application is conductivity in an X-Y plane of the electrode and transverse conductivity is conductivity in a Z plane of the electrode.

The conductive adhesive may also have cutouts for controlling lateral conductivity of the electrode and the member cutouts and adhesive cutouts may be aligned with one another or offset depending upon the desired resistivity/conductivity patterns required across the electrode.

As will be hereinafter described, the cutouts may be symmetric or asymmetric in shape.

Still another embodiment of the present invention is directed to a medical electrode as hereinabove described but utilizing a connector which includes a leadwire interconnecting conductive patterns with the leadwire having conductive portions in electrical communication with the patterns and non-conductive portions between the patterns.

### BRIEF DESCRIPTION OF THE DRAWINGS

The present invention may be better understood with reference to the following detailed description, taken in conjunction with the accompanying drawings, in which:

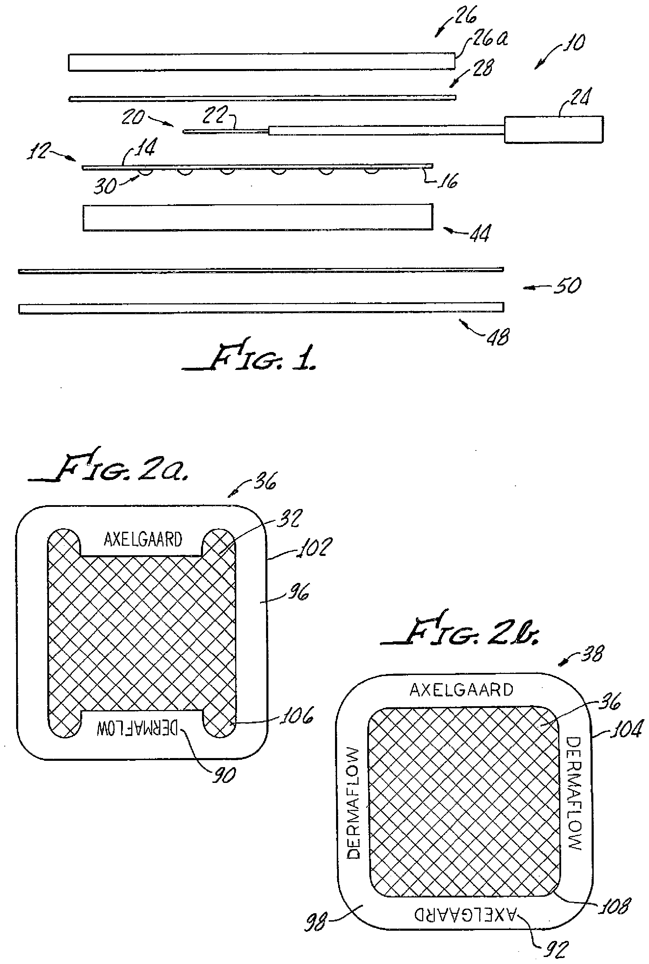

Figure 1 is an exploded cross-sectional view of a medical electrode in accordance with the present invention generally showing a moderately conductive flexible member having a top side and a bottom side, a connector in contact with the member top side, and a non-conductive flexible sheet covering the moderately conductive flexible member top side and the connector, the sheet having dimensions greater than the flexible member; a highly conductive pattern is disposed on the member bottom side, along with a moderately high conductive adhesive and a plastic carrier with a release layer, the carrier preventing premature and/or inadvertent contact with the hydrogel;
Figures 2a and 2b are plan views of two embodiments of the present invention showing different conductive ink patterns and indicia for identifying electrodes;
Figure 3 is a plan view of yet another embodiment of the present invention showing several conductive ink patterns disposed on a single conductive flexible member and a non-conductive flexible sheet extending beyond a perimeter of the member;
Figure 4 is a plot of current distribution profile of the electrode shown in Figure 1, (i.e. an electrode having a moderately conductive flexible member, a highly conductive pattern disposed on the member bottom side and a moderately high conductive adhesive);
Figure 5 is a plot similar to Figure 4, and included for comparison purposes, of the current distribution of an electrode having a moderately conductive flexible member, a highly conductive pattern disposed on the member bottom side and moderately conductive adhesive;
Figure 6 is a plot similar to Figure 5, and included for comparison purposes, of the current distribution of an electrode with no highly conductive pattern disposed on a moderately conductive member bottom side, a moderately conductive adhesive and a connector disposed on a top side of the moderately conductive member;
Figure 7 is an exploded cross-sectional view of another embodiment of the present invention, similar to the embodiment shown in Figure 1 but with cutouts in the conductive member and adhesive for controlling lateral conductivity within the electrode;
Figure 8 is a plan view of the electrode shown in Figure 7 more clearly showing conductive member and/or adhesive cutouts between conductive patterns;
Figure 9 is a plan view of yet another embodiment of the present invention utilizing conductive member and/or adhesive cutouts;
Figure 10 is an exploded cross-sectional view of a medical electrode in accordance with the present invention generally showing a conductive flexible member, conductive patterns as illustrated in Figure 1 but with a connector with a leadwire having conductive portions in electrical communication with the patterns and non-conductive portions between the patterns;
Figure 11 is a plan view of the electrode shown in Figure 10;
Figure 12 is an exploded cross-sectional view of an embodiment of the present invention similar to the embodiment shown in Figure 10 but with separated conductive flexible members, conductive patterns and adhesive layers; and
Figure 13 is a plan view of the electrode shown in Figure 12.

### DETAILED DESCRIPTION

With reference to Figure 1, there is shown, in exploded cross-section, a single medical electrode 10 in accordance with the present invention, which generally includes a moderately conductive flexible member, or sheet, 12 having a top side 14 and a bottom side 16.

Multiple electrodes are utilized in accordance with the present invention as will be hereinafter described in greater detail.

A connector 20, which may include a lead wire 22 and jack 24 is provided with the lead wire 22 in contact with the member top side 14.

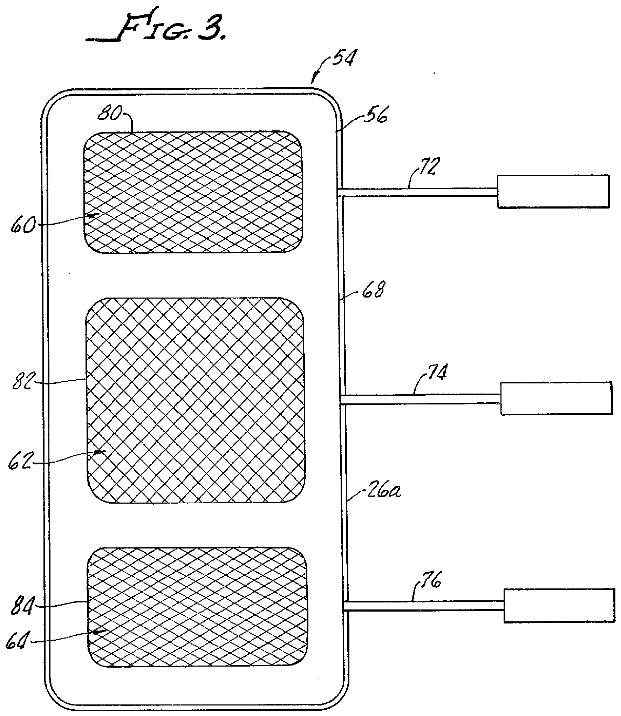

A non-conductive flexible oversize sheet 26 covers the conductive flexible member 12 along with the connector 20 in order to prevent inadvertent contact with the conductive member 12 and connector 20. The sheet 26 may be adhered to the flexible member 12 with any suitable adhesive 28 and also holds the lead wire 22 in contact with the member 12. The sheet 26 has dimensions greater than overall dimensions of the member 12 resulting in an edge, or perimeter, 26a which overlaps the member 12, see also Figure 3. This structure eliminates the need for alignment of the sheet 26 with the member along their peripheries. The adhesive 28 covers the entire sheet 26 include the edge, or perimeter, 26a. This enables the electrode 10 to be sealed along the perimeter 26a to a user's skin (not shown). This, in turn, enables bathing or showering by the user without degradation of the electrode/skin coupling. Water is prevented from entering the electrode 10 under the sheet 26.

The lead wire 22 may be of any inexpensive suitable conductive material.

The sheet 12 may be formed from any suitable carbon loaded elastomeric film or conductive plastic having suitable surface resistivity of between about 10² ohm/square and about 10⁶ ohm/square, for example, about 10⁵ ohm/square and a transverse resistivity of between about 10 and about 10⁴ ohm/square, for example, about 10² ohm/square. Suitable polycarbonate polyolefin and a conductive ink pattern 30 may be printed, or otherwise transferred to the conductive member bottom side 16 with various patterns 32, 34 for embodiments 36, 38, as shown in Figures 2a and 2b. The ink pattern may have a resistivity of between about 0.1 and about 10 ohm/cm.

With reference again to Figure 1, the conductive ink pattern 30 contacts a conductive hydrogel adhesive 44, which is utilized for adhering the electrode 10 to a patient's skin, not shown. The conductive hydrogel adhesive is formulated with moderately high conductivity for example a volume resistivity between about 10² and about 10⁴ ohm cm, preferably about 400 ohm cm. Suitable gels are described in U.S. 6,038,464.

A plastic, paper, or other suitable carrier 48 along with a release coating 50 may be provided in order to prevent inadvertent and/or premature adhesion of the patients' skin or other object to the hydrogel. The plastic carrier 48 and release coating 50 is removed prior to application of the electrode 10 to the patients' skin.

Shown in Figure 3 is another electrode embodiment 54 which includes a moderately conductive flexible member 56 having a plurality of highly conductive ink patterns 60, 62, 64 disposed on a bottom side 68 of the conductive member 56. The conductive ink patterns 60, 62 and 64 may be of various shapes and grid patterns in order to customize the electrical conductivity of the electrode 54 beneath the pattern 60, 62, 64. The adhesive, not shown in Figure 3, is of moderately high conductivity as hereinabove described.

The spaced apart patterns 60, 62 and 64 act as separate electrodes and communicate with lead wires, or connectors, 72, 74, 76 respectively, which are attached to a top side (not shown in Figure 3) of the conductive member 56 as illustrated in Figure 1 with the description of the electrode embodiment 10.

The advantage of utilizing a common conductive member 56 with spaced apart ink patterns 60, 62, 64 is that this structure provides uniformity of spacing between the independent electrodes. This in effect provides a template to insure proper electrode placement on a patient's skin.

It should be appreciated that, as shown in Figure 3, the connector 72, 74, 76 are in placed over the ink patterns 60, 62, 64. The lead wires 72, 74, 76 may be placed anywhere between the borders 80, 82, 84 of the ink patterns 60, 62, 64 since the current distribution across the electrode gel adhesive 44 is independently controlled as hereinabove noted.

Referring to Figures 2a and 2b, the borders 96, 98 are created when the conductive ink patterns 32, 34 are disposed on the flexible member bottom side 16 at a spaced apart distance from a perimeter 102, 104 of the conductive member 12. That is, a perimeter 106, 108 of the ink patterns 32, 34 is spaced apart from the perimeters 102, 104. Indicia 90, 92 may or may not be conductive ink, may be printed on the borders 96, 104 and no not interfere with the current density of the electrode 10.

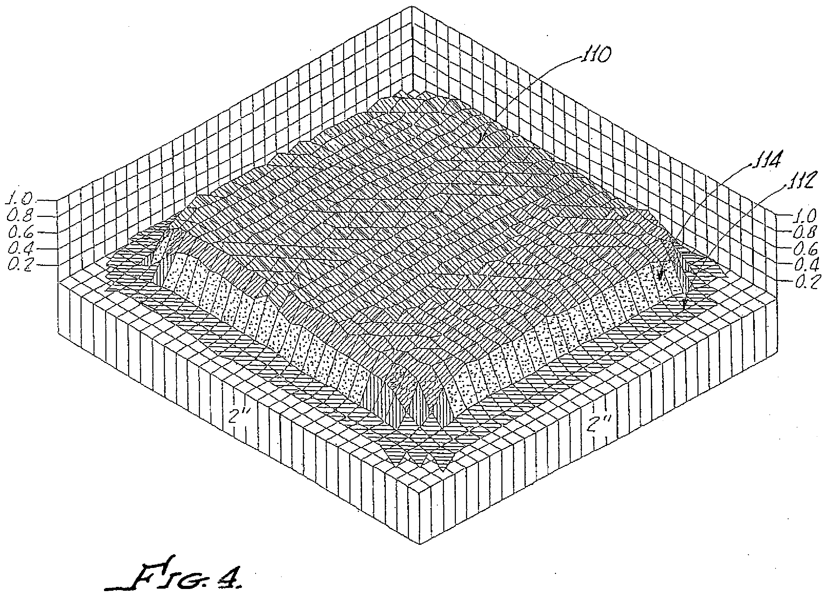

Figure 4 shows the current distribution profile for the electrodes shown in Figure 2 utilizing a flexible conductive member having a surface resistivity of about 200 ohm/square, a conductive ink pattern having conductivity of about 1 ohm/cm and a hydrogel such as set forth in U.S. Patent No. 6,038,464 with a volume resistivity of about 400 ohm cm. This patent describes this type of gel in general electrical configuration which may be used to advantage in accordance with the present invention.

As shown in Figure 4, the conductivity of a 2-inch (50 mm) square electrode is very uniform over almost the entire electrode surface with very little edge effects, i.e. perimeter edges in which non-uniform conductivity occurs, typical with prior art electrodes. In Figure 4, a center area 110 represents high current density or current transfer by the electrode, the peripheral area 112 represents low or no current density and an intermediate area 114 represents a sharp roll off of current density. Because of the rapid roll off in current density, the efficiency of the electrode is enhanced since most of the electrode is utilized for providing uniform current density without burning edge effects.

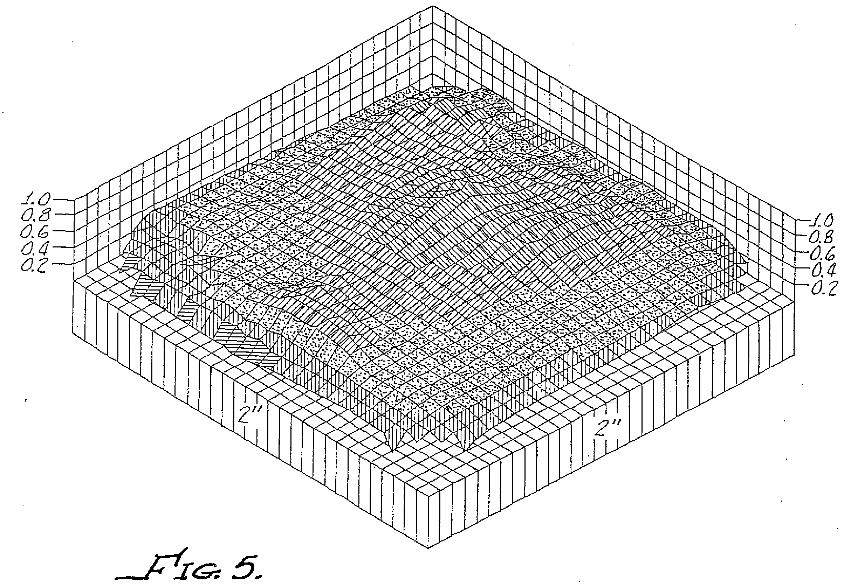

The current density plot of Figure 4 shows a vastly improved current density over the electrode in Figure 5 which is identical except for the use of a moderately conductive adhesive (about 1100 ohm cm) instead of a moderately high conductive adhesive (about 400 ohm cm).

It should be clear that the current density shown in Figure 5 is considerably more non-uniform than the current density shown in Figure 4. Accordingly, the electrode efficiency in coupling current to a patient (not shown) is severely diminished.

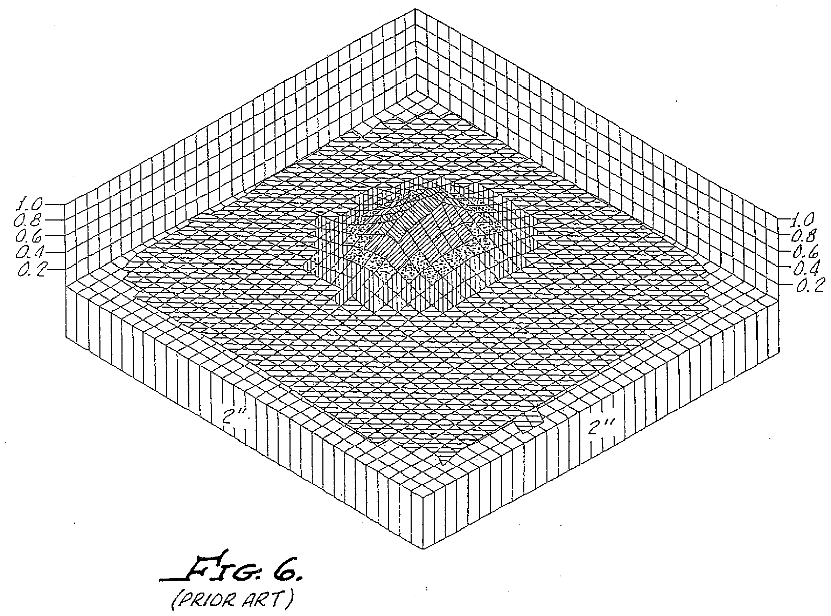

Figure 6 is a plot of current density of an electrode as constructed similar to the electrode of Figure 5 with a moderately conductive flexible member and a moderately conductive adhesive but with the lead wire disposed on top of the flexible member and no conductive pattern on the bottom side. The current density shown in Figure 6 (which is representative of prior art electrodes) is by far inferior (hot spot) to the current densities shown in Figure 4 and 5 and illustrates that the unique combination of elements collectively provides an electrode having unexpectedly improved current density.

The difference in conductivity or resistivity between the sheet 12 and the pattern 30 as well as the adhesive 44 enables precise control of current distribution which cannot be achieved, for example, with a non-conductive sheet or a highly conductive sheet. The conductivity of the adhesive is selected to be moderately high in order to enable the connector 20 to be disposed on top of the sheet 12 instead of in contact with the pattern as with prior art electrode. This effect of adhesive conductivity was heretofore not known and is an unexpected result. In addition, the ink pattern may be of varied conductivity in order to tailor the current through the conductive sheet which may have a thickness of up to about 10 mils, for example, about 1 mil.

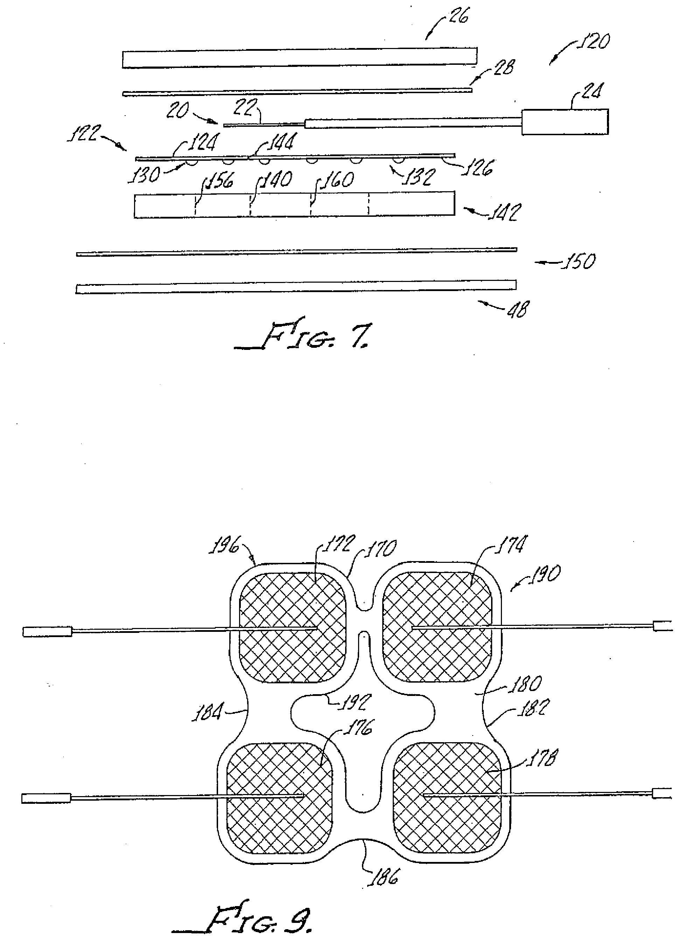

With reference to Figure 7, there is shown another embodiment 120 of the present invention with elements identical or substantially similar to the embodiment 10 shown in Figure 1 having the same reference characters.

In this embodiment 120, a moderately conductive flexible member 122 includes a top 124 and a bottom 126 with a plurality of highly conductive patterns 130, 132 disposed on the bottom side 126 of the conductive member 122. As hereinabove noted, the conductive patterns 130, 132 may be formed from conductive ink.

Lateral conductivity between the electrode patterns 130, 132 is controlled by a cutout 140 in a conductive adhesive layer 142 and/or a cutout 144 in the conductive flexible member 122. The cutouts also provide for improved flexibility and conformability of the embodiment 120. Lateral conductivity may also be controlled by varying a thickness of the conductive flexible member and/or conductive adhesive. In that regard, the thickness may not be uniform with variation in thickness used to control lateral conductivity.

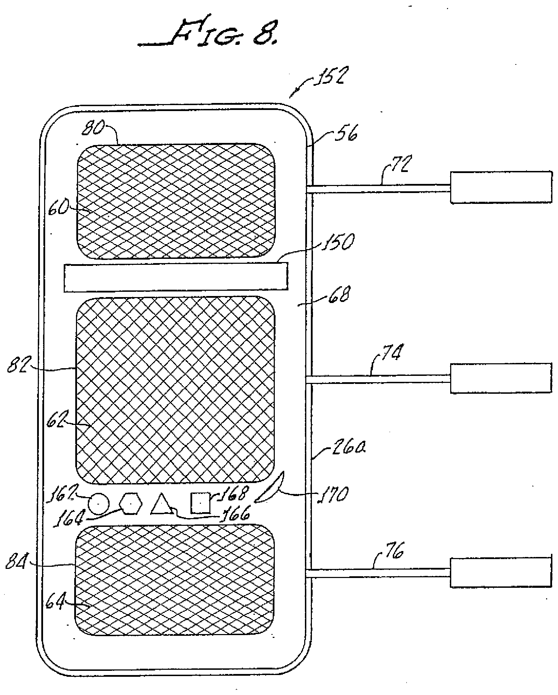

A similar cutout 150 is shown in Figure 8 in an embodiment 152, similar to the embodiment 54 shown in Figure 3 with identical or substantially similar elements being identified by common reference numbers.

While the cutout 150 is shown between conductive patterns 60, 62 in Figure 8, it should be appreciated that the cutout may be disposed under the pattern 130 as indicated by the dashed line 156 in Figure 7. In addition, while the cutout 140, 144 may be aligned in a transverse direction as illustrated in Figure 7, the cutouts may be laterally spaced apart, as indicated by the cutouts 144, 156 or partially overlap as indicated by a cutout line 160 with respect to the cutout 144, also shown in Figure 7.

The lateral conductivity of the electrode 152 as shown in Figure 8 may be controlled by cutouts 162, 164, 166, 168, 170 which may be of any symmetric or asymmetric shape and disposed for controlling the lateral conductivity as empirically determined.

The technology of the present invention is particularly useful in application specific electrodes, such as a highly conformable medical back electrode array 190 shown in Figure 9 which includes conductive patterns 172, 174, 176, 178 disposed on conductive flexible member 180, as hereinabove described with cutouts 184, 186, 182, 170, 192. This further illustrates the use of cutouts 184, 186, 182, 170, 192 on a perimeter 196 of the electrode 190. The spaced apart patterns 172, 174, 176, 178 are anatomically arranged in order to facilitate proper placement on a patient.

While the conductive patterns 172, 174, 176, 178 are preferably formed from conductive ink, it should be appreciated that the patterns 172, 174, 176, 178 as well as all other patterns herein described may be any conductive material which would include but not limit to meshes and solid components which may be formed from any conductive plastic or metal.

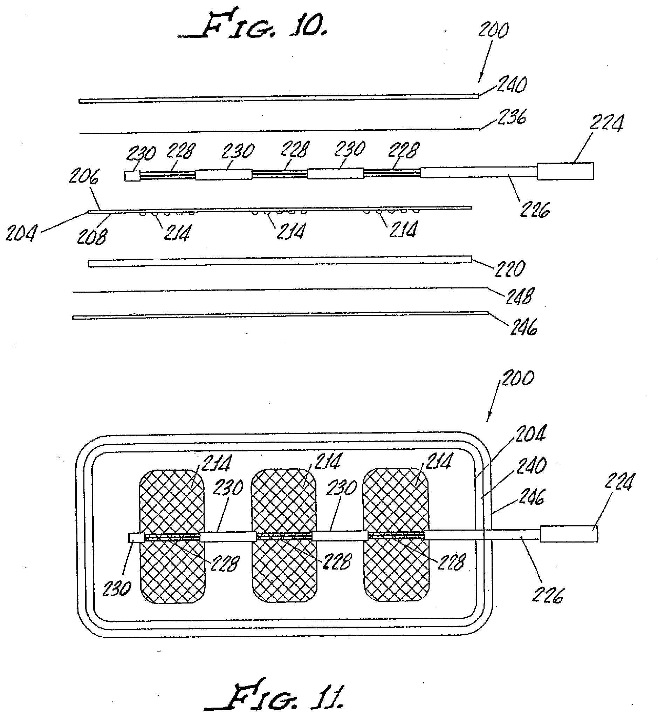

With reference now to Figures 10 and 11 there is shown an electrode 200 which is still another embodiment of the present invention which included elements similar to the embodiments hereinabove described.

The electrode 200 includes a moderately conductive flexible member 204, such as, for example, a carbon film, having a top side 206 and a bottom side 208. As hereinabove described a plurality of highly conductive patterns 214 disposed on the bottom side 208 of the conductive flexible member 204. The patterns are disposed in a spaced apart relationship, as shown, and while only three (3) patterns are shown, any suitable number may be used.

A moderately high conductive adhesive layer 220 is disposed on the conductive flexible member bottom side 208 and covers the conductive patterns 214 while also functioning to adhere the electrode 200 to a patient's skin, not shown.

A connector 224 provided for interconnecting the conductive patterns 214 establishes electrical contact with an external apparatus, not shown. The connector 224 includes a leadwire 226 which again includes conductive portions 228 for providing electrical communication with the patterns 214 and non-conductive portions 230 between the patterns 214 in order to isolate the patterns from the conductive flexible member 204.

The non-conductive portion, or insulation, 230 on an end of the leadwire 226 is optional. It is only needed if the strands of the leadwire 226 are stiff and protection against cutting through the member 204 and adhesive layer 220 is necessary.

The conductive portions 228 and non-conductive portions 230 may be prepared by any suitable fashion such as, for example, selective stripping, coating or attaching bands of insulation material.

A non-conductive flexible sheet, or backing 240, with an adhesive layer 236 may be disposed over the leadwire on the conductive flexible member top side 206 with an overlap of the conductive flexible member 204 by a perimeter 240 similar to the embodiments hereinabove described and a removable line 246 with a release layer 248 prevents contamination of the electrode 200 prior to use.

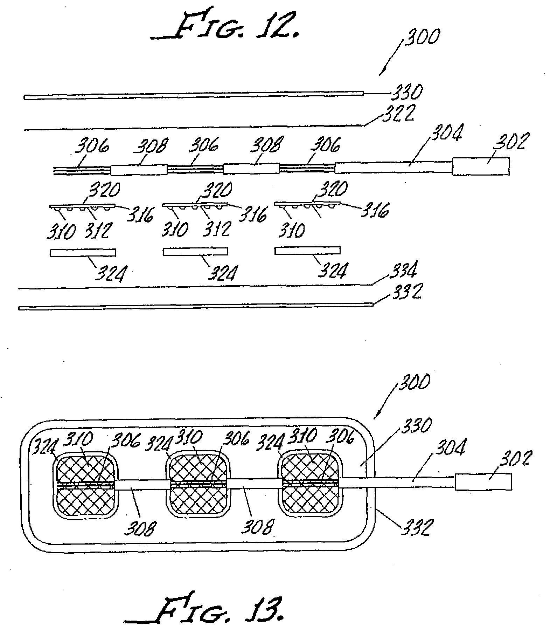

Figures 12 and 13 illustrate an electrode 300 in accordance with the present invention which also uses a conductor 302 having a leadwire 304 having conductive portions 306 and non-conductive portions 308, with the conductive portions 306 in electrical communication with spaced apart highly conductive patterns 310 disposed on bottom sides 312 of a plurality of moderately conductive flexible members 316, the electrical communication occurring through a top side 320 of each of the conductive flexible members 316.

The plurality of conductive patterns are covered by a plurality of moderately high conductive adhesive layers 324 which serve to adhere the electrode 300 and conductive patterns 310 to a patient's skin, not shown.

A non-conductive flexible sheet 330 is disposed over the leadwire 304 on the conductive flexible member top side 320 and the components are bonded by an adhesive layer 322. In addition, as hereinabove described a line 332 with a release layer 334 is provided to prevent contamination of the electrode 300 prior to use.

Although there has been hereinabove described a specific electrode in accordance with the present invention for the purpose of illustrating the manner in which the invention may be used to advantage, it should be appreciated that the invention is not limited thereto. That is, the present invention may suitably comprise, consist of, or consist essentially of the recited elements. Further, the invention illustratively disclosed herein suitably may be practiced in the absence of any element which is not specifically disclosed herein. Accordingly, any and all modifications, variations or equivalent arrangements which may occur to those skilled in the art, should be considered to be within the scope of the present invention as defined in the appended claims.

## Claims

1. A medical electrode (200, 300) comprising:
a moderately conductive flexible member (204, 316) having a top side and a bottom side, the moderately conductive flexible member comprising a surface resistivity between 10² and 10⁶ ohm/cm;
a plurality of highly conductive patterns (214, 310) disposed on the moderately conductive flexible member bottom side in a spaced apart relationship, the plurality of highly conductive patterns comprising a surface resistivity between 0.1 and 10² ohm/cm;
a moderately highly conductive adhesive layer (220, 324) disposed on the conductive flexible member bottom side and covering said plurality of conductive patterns for adhering the medical electrode to a patient's skin, the moderately highly conductive adhesive layer comprising a volume resistivity between 10² and 10⁴ ohm cm; and
a single connector (224, 302), including a common leadwire (226, 304) interconnecting each of the plurality of highly conductive patterns, the single connector configured to electrically connect with an external apparatus, **characterised in that** the common leadwire is configured to have conductive portions (228, 306) in electrical communication with the plurality of highly conductive patterns and non-conductive portions between the plurality of highly conductive patterns.

2. The electrode according to claim 1 wherein the common leadwire is disposed on the moderately conductive flexible member top side.

3. The electrode according to claim 2 wherein the common leadwire conductive portions are aligned with the plurality of highly conductive patterns.

4. The electrode of claim 3, including non-conductive portions (230, 308) disposed on the common leadwire between the plurality of highly conductive patterns.

5. The electrode of claim 4, wherein the moderately highly conductive adhesive layer comprises a plurality of moderately highly conductive adhesive layers (324) with cutouts between each of the plurality of moderately highly conductive adhesive layers.

6. The electrode of claim 5, wherein the plurality of moderately highly conductive adhesive layers are aligned with the plurality of highly conductive patterns.

7. The electrode of claim 6, wherein the moderately conductive flexible member comprises carbon film.

8. The electrode of claim 7, wherein the non-conductive portions of the common leadwire comprises an insulative coating, a band of insulation, or a selective insulative stripping aligned between the plurality of highly conductive patterns.

9. The electrode of claim 8, including a non-conductive flexible sheet with a non-conductive adhesive layer disposed over the common leadwire and moderately conductive flexible member.

10. The electrode of claim 9, wherein the non-conductive flexible sheet and non-conductive adhesive layer are larger in size than the moderately conductive flexible member and comprise a perimeter overlap that is configured to adhere to the patient's skin.

## Patentansprüche

1. Medizinische Elektrode (200, 300) umfassend:
ein mäßig leitfähiges flexibles Element (204, 316) mit einer oberen Seite und einer unteren Seite, wobei das mäßig leitfähige flexible Element einen spezifischen Oberflächenwiderstand von zwischen 10² und 10⁶ Ohm/cm aufweist;
eine Vielzahl von hochleitfähigen Mustern (214, 310), die auf der unteren Seite des mäßig leitfähigen flexiblen Elements in einem Abstand voneinander angeordnet sind, wobei die Vielzahl der hochleitfähigen Muster einen spezifischen Oberflächenwiderstand von zwischen 0,1 und 10² Ohm/cm aufweist;
eine mäßig hochleitfähige Haftschicht (220, 324), die an der unteren Seite des leitfähigen flexiblen Elements angeordnet ist und die Vielzahl von leitfähigen Mustern bedeckt, so dass die medizinische Elektrode an der Haut eines Patienten haften kann, wobei die mäßig hochleitfähige Haftschicht einen spezifischen Volumenwiderstand von zwischen 10² und 10⁴ Ohm/cm aufweist, und
einen einzelnen Verbinder (224, 302) mit einem gemeinsamen Zuleitungsdraht (226, 304), der die Vielzahl von hochleitfähigen Mustern jeweils miteinander verbindet, wobei der einzelne Verbinder so ausgebildet ist, dass er mit einer externen Vorrichtung elektrisch verbunden werden kann, **dadurch gekennzeichnet, dass** der gemeinsame Zuleitungsdraht so ausgebildet ist, dass die leitenden Abschnitte (228, 306) mit der Vielzahl von hochleitfähigen Mustern und nicht leitfähigen Abschnitten zwischen der Vielzahl von hochleitfähigen Mustern in elektrischer Verbindung stehen.

2. Elektrode nach Anspruch 1, wobei der gemeinsame Zuleitungsdraht an der oberen Seite des mäßig leitfähigen flexiblen Elements angeordnet ist.

3. Elektrode nach Anspruch 2, wobei die leitenden Abschnitte des gemeinsamen Zuleitungsdrahts mit der Vielzahl von hochleitfähigen Mustern ausgerichtet sind.

4. Elektrode nach Anspruch 3 mit nicht leitfähigen Abschnitten (230, 308), die an dem gemeinsamen Zuleitungsdraht zwischen der Vielzahl von hochleitfähigen Mustern angeordnet sind.

5. Elektrode nach Anspruch 4, wobei die mäßig hochleitfähige Haftschicht eine Vielzahl von mäßig hochleitfähigen Haftschichten (324) mit Ausschnitten zwischen jeder der Vielzahl von mäßig hochleitfähigen Haftschichten aufweist.

6. Elektrode nach Anspruch 5, wobei die Vielzahl von mäßig hochleitfähigen Haftschichten mit der Vielzahl von hochleitfähigen Mustern ausgerichtet ist.

7. Elektrode nach Anspruch 6, wobei das mäßig leitfähige flexible Element einen Kohlenstofffilm aufweist.

8. Elektrode nach Anspruch 7, wobei die nicht leitfähigen Abschnitte des gemeinsamen Zuleitungsdrahts eine isolierende Beschichtung, ein Isolationsband oder eine selektive Abisolierung aufweisen, die zwischen der Vielzahl von hochleitfähigen Mustern ausgerichtet sind.

9. Elektrode nach Anspruch 8 mit einer nicht leitfähigen flexiblen Folie mit einer nicht leitenden Haftschicht, die über dem gemeinsamen Zuleitungsdraht und dem mäßig leitfähigen flexiblen Element angeordnet ist.

10. Elektrode nach Anspruch 9, wobei die nicht leitfähige flexible Folie und die nicht leitfähige Haftschicht größer sind als das mäßig leitfähige flexible Element und eine Umfangsüberlappung haben, die so ausgebildet ist, dass sie an der Haut des Patienten haftet.

## Revendications

1. Electrode médicale (200, 300) comprenant :
un élément flexible modérément conducteur (204, 316) ayant un côté supérieur et un côté inférieur, lequel élément flexible modérément conducteur présente une résistivité surfacique comprise entre 10² et 10⁶ ohm/cm ;
une pluralité de motifs hautement conducteurs (214, 310) disposés sur le côté inférieur de l'élément flexible modérément conducteur dans une relation espacée, laquelle pluralité de motifs hautement conducteurs présente une résistivité surfacique comprise entre 0,1 et 10² ohm/cm ;
une couche adhésive modérément hautement conductrice (220, 324) disposée sur le côté inférieur de l'élément flexible conducteur et recouvrant ladite pluralité de motifs conducteurs pour faire adhérer l'électrode à la peau d'un patient, laquelle couche adhésive modérément hautement conductrice présente une résistivité volumique comprise entre 10² et 10⁴ ohm cm ; et
un connecteur unique (224, 302) comprenant un fil de connexion commun (226, 304) interconnectant chacun de la pluralité de motifs hautement conducteurs, lequel connecteur unique est configuré pour établir une connexion électrique avec un appareil externe,
**caractérisée en ce que** le fil de connexion commun est configuré pour avoir des parties conductrices (228, 306) en communication électrique avec la pluralité de motifs hautement conducteurs et des parties non conductrices entre la pluralité de motifs hautement conducteurs.

2. Electrode selon la revendication 1, dans laquelle le fil de connexion commun est disposé sur le côté supérieur de l'élément flexible modérément conducteur.

3. Electrode selon la revendication 2, dans laquelle les parties conductrices du fil de connexion commun sont alignées avec la pluralité de motifs hautement conducteurs.

4. Electrode selon la revendication 3, comprenant des parties non conductrices (230, 308) disposées sur le fil de connexion commun entre la pluralité de motifs hautement conducteurs.

5. Electrode selon la revendication 4, dans laquelle la couche adhésive modérément hautement conductrice comprend une pluralité de couches adhésives modérément hautement conductrices (324) avec des découpes entre chacune de la pluralité de couches adhésives modérément hautement conductrices.

6. Electrode selon la revendication 5, dans laquelle la pluralité de couches adhésives modérément hautement conductrices sont alignées avec la pluralité de motifs hautement conducteurs.

7. Electrode selon la revendication 6, dans laquelle l'élément flexible modérément conducteur comprend un film de carbone.

8. Electrode selon la revendication 7, dans laquelle les parties non conductrices du fil de connexion commun comprennent un revêtement isolant, une bande d'isolation ou un dénudage isolant sélectif aligné entre la pluralité de motifs hautement conducteurs.

9. Electrode selon la revendication 8, comprenant une feuille flexible non conductrice avec une couche adhésive non conductrice disposée sur le fil de connexion commun et l'élément flexible modérément conducteur.

10. Electrode selon la revendication 9, dans laquelle la feuille flexible non conductrice et la couche adhésive non conductrice sont plus grandes en taille que l'élément flexible modérément conducteur et comprennent un débordement périphérique qui est configuré pour adhérer à la peau du patient.
